(19) Europäisches Patentamt
European Patent Office
Office européen des brevets

(11) Veröffentlichungsnummer: **0 034 375**
**B1**

(12) # EUROPÄISCHE PATENTSCHRIFT

(45) Veröffentlichungstag der Patentschrift: **22.08.84**

(21) Anmeldenummer: **81101156.8**

(22) Anmeldetag: **18.02.81**

(51) Int. Cl.³: **A 61 L 17/00**, C 08 L 83/08, C 08 L 83/06, C 08 L 83/04, C 07 F 7/21, C 07 F 7/12, C 07 F 7/08

(54) **Silikonkautschuk-Mischung zur therapeutischen Anwendung und als Heilbehelf.**

(30) Priorität: **18.02.80 HU LA000369**

(43) Veröffentlichungstag der Anmeldung: **26.08.81 Patentblatt 81/34**

(45) Bekanntmachung des Hinweises auf die Patenterteilung: **22.08.84 Patentblatt 84/34**

(84) Benannte Vertragsstaaten: **BE CH DE FR GB IT LI LU NL SE**

(56) Entgegenhaltungen:
**GB - A - 2 016 482**
**US - A - 3 056 756**
**US - A - 3 361 700**

**CHEMICAL ABSTRACTS, Band 87, Nr. 16, 17. Oktober 1977, Seite 30, Nr. 118495q Columbus, Ohio, U.S.A. K.A. ANDRIANOV et al.: "Reactivity of functional groups at silicon atoms in the heterofunctional polycondensation of organocyclosiloxanes with alpha, omega-poly(dimethylsiloxanes)"**

(73) Patentinhaber: **Lazar, Laszlo, Dr., Rozsahegy utca. 4, H-1024, Budapest (HU)**
Patentinhaber: **Palossy, Katalin, geb. Becker, Dr., Vinceller utca 31, H-1113 Budapest (HU)**
Patentinhaber: **Nagy, Jozsef, Dr., Törökbalinti, ut 46/b, H-1122 Budapest (HU)**
Patentinhaber: **Pasztor, Emil, Dr., Rakoczi ut 4., H-1072 Budapest (HU)**

(72) Erfinder: **Lazar, Laszlo, Dr., Rozsahegy utca. 4, H-1024, Budapest (HU)**
Erfinder: **Palossy, Katalin, geb. Becker, Dr., Vinceller utca 31, H-1113 Budapest (HU)**
Erfinder: **Nagy, Jozsef, Dr., Törökbalinti, ut 46/b, H-1122 Budapest (HU)**
Erfinder: **Pasztor, Emil, Dr., Rakoczi ut 4., H-1072 Budapest (HU)**

(74) Vertreter: **von Füner, Alexander, Dr. et al, Patentanwälte v. Füner, Ebbinghaus, Finck Mariahilfplatz 2 & 3, *D-8000 München 90 (DE)***

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 034 375 B1

ACTORUM AG

**Beschreibung**

Gegenstand der Erfindung ist eine neue Silikonkautschuk-Mischung zur therapeutischen Anwendung, sowie ein diese Mischung enthaltender Heilbehelf. Die neue Mischung und der diese Mischung enthaltende Heilbehelf können vor allem in der Gefässchirurgie des Gehirns bzw. in der allgemeinen Chirurgie zur operativen Verschliessung mittels Katheter eingesetzt werden.

Die erfindungsgemässe Silikonkautschuk-Mischung besteht aus zwei, bzw. drei Komponenten, deren eine einen Röntgenschatten gibt. Der erfindungsgemässe Heilbehelf besteht aus der obigen Silikonkautschuk-Mischung, dem zur Polymerisierung der Silikonkautschuk-Mischung benötigten Katalysator, sowie aus einem, zur Einführung in die Gehirnarterien und in andere Blutgefässe, bzw. Drüsenwege geeigneten, gegebenenfalls mit einem Aufblasballon versehenen Mikrokatheter.

Die Anwendung der obengenannten neuen Silikonkautschuk-Mischung bzw. des Heilbehelfs für verschiedene therapeutische Zwecke, insbesondere bei Gehirnoperationen, bzw. in der Gefässchirurgie und auf den Gebieten der allgemeinen Chirurgie bildet ebenfalls den Gegenstand der Erfindung.

Gewisse Bereiche des menschlichen arteriellen Gefäss-Systems sind wegen ihrer Lage oder Grösse (Arterien zweiter oder dritter Ordnung) weder durch direkten chirurgischen Eingriff, noch endovasculär, das heisst mit dem üblichen steifen Katheter vom sog. Seldingerschen System zugänglich. Dieses Problem wurde durch die Entwicklung der mit einem kleinen, aufblasbaren Ballonkopf aus Naturkautschuk versehenen, die Treibkraft des Blutstroms benützenden, flexiblen Ballonkatheter mit einem Durchmesser unter 1 mm gelöst, die insbesondere in der Gefässchirurgie des Gehirns von grundlegender Bedeutung sind.

Die Gehirnadern sind an der Schädelbasis von steifen Knochenwänden begrenzt, und voller Windungen (Syphone). Daher können diese zerebralen Aderabschnitte nur mit den neuen Kathetern erreicht werden. Das in den Mikrokatheter eingeführte Röntgenkontrastmittel ermöglicht unter Anwendung eines Bildverstärkers die genaue Verfolgung der Position des Katheters, während der Gebrauch mehrerer Katheter auch den Zugang zu den hinsichtlich der Strömung sekundären Gefässabschnitten gewährleistet. Das genannte Verfahren ist aber nur von diagnostischer Bedeutung; wird nämlich der in die Zielstelle eingeführte Katheter in der Ader gelassen, so verschliesst dieser - infolge einer sekundären Thrombose — auch die funktionell wichtigen Hauptgefässe. Diese Komplikation kann vermieden werden, indem man den Ballonkopf unter Abtrennung und Zurückziehung des für das Einführen notwendigen Katheterstiels isoliert in der Ader zurücklässt. Die Abtrennung, somit die sog. superselektive Embolisierung beruht auf der physikalischen Tatsache, dass der aufgeblasene Ballonkopf an der inneren Wand des zu verschliessenden Aderabschnitts mit einer mechanischen Kraft haftet, die grösser ist, als die Kraft der elastischen Verbindung zwischen dem Ballon und dem Katheterende. Die Abtrennung des mit Flüssigkeit gefüllten Ballonkopfes ist wegen der möglichen Verschliessung des Ballons teils gefährlich, teils kann sie erfolglos sein, denn nach der Abtrennung strömt die Flüssigkeit aus dem Ballon durch die Endöffnung hinaus. Wird nun in den Katheter eine flüssige, aber in kurzer Zeit fest werdende Substanz eingeführt, so ermöglicht dies die stabile Fixierung des Ballons im Gefäss und die endgültige, sichere Verschliessung an der gewünschten Stelle.

Aufgabe der Erfindung ist die Entwicklung eines neuen und vorteilhaften Heilbehelfs, mit dessen Hilfe die oben geschilderte Abtrennung und dadurch die Embolisierung einzelner Adern, bzw. die Verschliessung sonstiger Wege leicht und mit grosser Sicherheit durchgeführt werden kann, ohne dass beim Abtrennen des Katheters Probleme auftauchen.

Das auf dem Gebrauch eines Ballonkatheters beruhende Verfahren wurde in den vergangenen Jahren im Prinzip von R. Scherbinenko ausgearbeitet (J. of Neurosurgery 41, 125-145 [1974]), er fand aber keinen geeigneten Stoff zur Abtrennung des Katheters, darüber hinaus konnte der Katheter mangels Kontrastmittel nicht verfolgt werden.

P. Schaps beschreibt zwar in seinem Artikel (Zentralblatt für Neurochirurgie 38, 105-19 [1977]) unter Anwendung eines Mikrokatheters und eines Ballons das Einsetzen von Silikon, allein die hohe Viskosität des gewählten Stoffes benötigte die Anwendung einer Eiskühltechnik, zudem gab er keinen Röntgenkontrast.

G. Debrun, P. Lacour, J. Caron und Mitarbeiter (J. of Neurosurgery 49, 635-49 [1978]) berichten von einer ähnlichen Methode, aber zugleich von Schwierigkeiten bei der Abtrennung. Bei chirurgischen Eingriffen war die Abtrennung nur mit Hilfe eines äusseren (koaxialen) Katheters möglich, somit war das Verfahren für Gehirnoperationen unbrauchbar.

J. K. Hilel, P. Sane, W. J. Michelson und A. Kosseim, Neuroradiology 16, 430-33 (1978) beschreiben in ihrem Artikel die Anwendung für die Mikrokatheter-Ballon-Technik eines Gemisches von einem Silikon-Elastomer (Silastic 382), von Methylsilikonöl und Tantalpulver. Die Nachteile dieses Gemisches bestehen teils in der hohen Viskosität, die die Einspritzung erschwert, teils darin, dass das Tantalpulver wegen Klumpenbildung den Katheter verstopft, zudem wirkt das Tantalpulver toxisch. Die Silikonkautschuk-Mischung war nach Vulkanisierung zur selektiven Abtrennung nicht geeignet.

Aus dem Gesagten geht hervor, dass bisher kein allen Anforderungen entsprechendes Mittel zur Verfügung stand. Die zur Anwendung der beschriebenen Methode geeignete Substanz muss nämlich folgenden Anforderungen genügen:

1. Von wesentlicher Bedeutung ist die niedrige Viskosität; der Stoff muss nämlich durch einen Katheter von 150 cm Länge und 0,1 mm innerem Durchmesser leicht befördert werden können. Zieht man in Betracht, dass nachher die eingepresste Flüssigkeit in einer verhältnismässig kurzen Zeit einen festen Zustand aufnehmen muss, so ist dies eine äusserst schwer zu lösende Aufgabe.

2. Angesichts der operativen Belastung des Patienten soll die Substanz binnen 10–20 Minuten fest werden, dabei muss zum Injizieren genügend Arbeitszeit (zumindest 3–4 Minuten) zur Verfügung stehen.

3. Der Stoff soll den Innenraum des Katheters gleichmässig ausfüllen, und während der Einspritzung keine Luftblasen bilden.

4. Er muss zugleich in festem Zustand gewissermassen unelastisch sein und an der Stelle der Abtrennung des Ballonkopfes leicht brechen.

5. Um die genaue Dosierung und die Kontrolle zu ermöglichen, muss die Substanz zugleich einen Röntgenschatten geben, mit dessen Hilfe die Position des Katheters und die genaue Stelle des Ballons verfolgt werden können.

Die wichtigste der angeführten Forderungen ist diejenige des verlässlichen Abbrechens (Punkt 4).

Sämtlichen obengenannten Anforderungen genügt die von uns entwickelte Silikonkautschuk-Mischung die im wesentlichen die Komponente A des Heilbehelfs laut Erfindung bildet.

Die erfindungsgemässe Aufgabenstellung wird wie aus den nachstehenden Ansprüchen ersichtlich gelöst. Demgemäss ist Gegenstand der Erfindung eine zur Anwendung in der endovasculären und allgemeinen Chirurgie geeignete, vorteilhaft einen Röntgenschatten gebende Silikonkautschuk-Mischung, die dadurch gekennzeichnet ist, dass sie aus einem niederviskosen linearen Polysiloxan, nämlich einem Dialkyl-, Alkylaryl-, Alkenylalyl- oder Diarylpolysiloxan mit Hydroxyl-Alkoxy-, Acyloxy oder Amino-Gruppen als reaktiven Endgruppen, einem niederviskosen cyclischen Diniederalkyl-polysiloxan und/oder aus einem für den menschlichen Organismus unschädlichen siliziumorganischen oder nicht metallischen jodhaltigen organischen Röntgenkontraststoff, sowie gegebenenfalls aus einem Methylsilikon besteht, wobei die Mischung eine Viskosität von 10 bis 100 mPas besitzt.

Nach einer bevorzugten Ausführungsform enthält sie als lineares Polysiloxan das lineare Dimethyl-polysiloxan-α, ω-diol.

Es wird weiterhin bevorzugt, dass sie als cyclisches Diniederalkyl-polysiloxan Octamethyl-cyclo-tetrasiloxan enthält.

Vorzugsweise soll die Mischung als Kontraststoff Bis-jodmethyltetramethyl-disiloxan enthalten.

Die erfindungsgemässe Silikonkautschuk-Mischung besteht also aus einem von zwei verschiedenen flüssigen Polysiloxankomponenten in geeignetem Verhältnis zusammengestellten Gemisch und gegebenenfalls aus einem für den menschlichen Organismus unschädlichen Kontraststoff.

a) Die erfindungsgemässen linearen Polysiloxane können mit der allgemeinen Formel I charakterisiert werden,

$$x\text{-}Si(R)(R_1)\text{-}[O\text{-}Si(R)_2]_n\text{-}O\text{-}Si(R)(R_1)\text{-}x \qquad (I)$$

wobei x einer Hydroxyl-, Alkoxy, Acyloxy- oder Aminogruppe entspricht und R und $R_1$, jeweils einen Alkyl-, Alkenyl- oder Arylrest bedeuten.

Das besonders bevorzugte lineare Dimethylpolysiloxan-α, ω-diol, das sog. LMS, hat die folgenden physikalischen Konstanten:

$\overline{n}$ = 80–85
$\overline{M}$ = 6000–7000
$d_4^{25\,°C}$ = 0,976 g/cm³
$n_D^{25\,°C}$ = 1,4043
$\eta^{25\,°C}$ = 80–100 m.Pa.s (cP)

b) Das cyclische Diniederalkylpolysiloxan mit niedriger Viskosität kann z.B. das sog. $D_4 = (R_2SiO)_4$ oder $D_5 = (R_2SiO)_5$ sein.

Die besonders bevorzugte Verbindung $D_4$ der Zusammensetzung $[(CH_3)_2SiO]_4$, d.h. das Oktamethyl-cyclo-tetrasiloxan hat die folgenden physikalischen Konstanten:

M = 296
Sp. = 175 °C/0,1 MPa
$d^{20\,°C}$ = 0,9558 g/cm³
$n_D^{20\,°C}$ = 1,3968
$\eta^{20\,°C}$ = 2 m.Pa.s (cP)

Die Viskosität des Gemisches von linearem und cyclischem Polysiloxan ist der gewünschten Anwendung gemäss zwischen den Werten $\eta^{25\,°C}$ = 10 und 100 mPas eingestellt.

c) Gegebenenfalls enthält die Mischung auch ein Methylsilikonöl mit der Viskosität zwischen 5 und 20 mPas, um eine Viskositätsreduktion zu erzielen.

d) Die der obigen Mischung gegebenenfalls beigegebene flüssige Kontrastsubstanz kann eine für den menschlichen Organismus unschädliche silizium-organische Verbindung sein, die das kontrastierende Jodatom (oder Jodatome bzw. $C^{14}$ oder ein Jodisotop) im Molekül eingebaut enthält. In der erfindungsgemässen Mischung wird vorzugsweise das Bis-jodmethyl-tetramethyl-disiloxan verwendet, das der Formel II entspricht.

$$J\text{-}CH_2\text{-}Si(CH_3)_2\text{-}O\text{-}Si(CH_3)_2\text{-}CH_2\text{-}J \qquad (II)$$

Die physikalischen Konstanten dieser Verbindung sind die folgenden:

M = 414
Sp. = 134 °C/1333,22 Pa
$d_4^{20}$ = 1,1772 g/cm³
$n_D^{20}$ = 1,5263

Vorteilhaft enthält die Verbindung ein Jodisotop, zweckmässig $J^{131}$.

Falls auch Mischungen von höherer Viskosität, d.h. Gemische mit einer Viskosität von 100 mPas eingesetzt werden können, ist es möglich, auch von den in der angiologischen Diagnostik verwendeten, extrahierten, festen, auf geeignete Korngrösse verfeinerten jodhaltigen organischen Röntgenkontraststoffen – wie es z.B. die im Handelsverkehr befindlichen Amipaque, Uromiro u.a.m. sind – Gebrauch zu machen, die aber den angeführten Komponenten nur sorgfältig homogenisiert hinzugegeben werden dürfen. Die Anwendung dieser Stoffe ist jedoch schwieriger und erfordert mehr Aufmerksamkeit, denn die Korngrösse muss der Aufgabe stets vorher angepasst werden in der Weise, das die Teilchen bei der Einspritzung der Mikrokatheter nicht verstopft und sie weiterhin bei freier Embolisierung nicht durch das kapillare Gefässystem gelangen.

Die erfindungsgemässe Silikonkautschuk-Mischung, d.h. die Komponente A des Heilbehelfs enthält in jedem Fall das in Punkt a) beschriebene lineare Polysiloxan, während sie vor den Komponenten unter b) und d) mindestens eine enthalten muss.

Der Heilbehelf, bzw. die erfindungsgemässe Garnitur enthält neben der oben beschriebenen Komponente A noch die sog. Komponente B, d.h. einen für beliebige medizinische Zwecke verwendbaren, zur Vernetzung kalt vulkanisierbarer Kautschukarten geeigneten Katalysator, der für 8–10 Minuten den gewünschten Flüssigkeitsgrad und eine 15–25 Minuten dauernde Bindungszeit sichert.

Im Laufe unserer Versuche wurde festgestellt, dass bei den vorgeschriebenen Bedingungen der Wärmesterilisierung (30 Minuten bei 120°C) beide Komponenten ihre ursprünglichen chemischen und bakteriologischen Eigenschaften sowie ihr Vernetzungsvermögen behalten.

Ein weiterer Bestandteil des erfindungsgemässen Heilbehelfs ist der Mikrokatheter. Der Mikrokatheter kann der beabsichtigten Anwendung gemäss ein- oder mehrgängig sein.

Der Heilbehelf laut Erfindung kann ausser den Komponenten A und B sowie den Mikrokatheter auch einen Ballonkopf, gewöhnlich aus Naturkautschuk enthalten. Dieser Ballonkopf ist vor allem bei der endovasculären Embolisierung von Bedeutung, wo die in den Ballon eingespritzte Silikonkautschuk-Komponente auf Wirkung des Katalysators vulkanisiert wird und gleichsam einen Pfropf zur Verschliessung der betreffenden Ader bildet. Bei der sog. freien Embolisierung ist der Gebrauch des Ballonkopfes nicht unbedingt nötig, falls der gewünschte Gefässabschnitt auch ohne dies erreicht werden kann.

Bei der Anwendung des Heilbehelfs, bzw. der Garnitur wird der Mikrokatheter, an dessen Ende eventuell der Ballon angebracht ist, in den gewünschten Ader- oder sonstigen Gefässabschnitt eingeführt, sodann werden die Komponenten A und B sorgfältig, mindestens eine halbe Minute lang homogen vermischt, wonach die benötigte Menge in eine geeichte Tuberkulinspritze aufgesaugt und unter Kontrolle eines Bildverstärkers sofort in den Katheter eingespritzt wird. Sobald der restliche Stoff einen brechbar festen Zustand erreicht, wird der Katheterstiel mit einer leichten, drehenden Ziehbewegung vom Ballonkopf getrennt, nachher wird der Katheter mit dem einvulkanisierten Silikonkautschuk aus dem Gefäss zurückgezogen.

Die neue Silikonkautschuk-Mischung wurde in erster Linie für die zerebrale endovasculäre Operationstechnik entwickelt. Die Mischung bzw. der Heilbehelf können aber in allen Fällen eingesetzt werden, wo ein äusserer chirurgischer Eingriff unmöglich, bzw. die direkte chirurgische Erschliessung der betroffenen Ader kontraindiziert ist.

Die wichtigsten Indikationsgebiete der superselektiven endovasculären Embolisierung mit Ballonkatheter sind die folgenden:

1. Verschliessung arteriovenöser Fisteln;
2. Embolisierung arteriovenöser Angiome;
3. Embolisierung der pathologisch geaderte, hämophile Geschwülste nährenden Gefässzweige in unmittelbarer Nähe der Geschwulst, um deren chirurgische Entfernung zu erleichtern;
4. Isolierte Verschliessung saccülärer Gefässaneurismen;
5. Verschliessung des Systems der Ausgangswege exokriner Drüsen, vor allem derjenigen der Speicheldrüsen.

Wie bereits erwähnt, kann die Embolisierung durch den Einbau des Ballons in den zu verschliessenden Ader- oder sonstigen Gefässabschnitt ausgeführt werden. Es ist aber auch möglich, eine sog. freie Embolisierung vorzunehmen, wobei man das Gemisch der Komponenten A und B laut Erfindung unmittelbar bei der Applikationsstelle direkt in den Gefässabschnitt, bzw. in den zu verschliessenden angiomatösen Bereich injiziert und der Vulkanisationsprozess hier verläuft. Anhand der Versuche wurde festgestellt, dass die Mischung laut Erfindung weder im ursprünglichen Zustand, noch im Laufe der Vulkanisierung eine toxische Wirkung ausübt.

Der Embolisierungsstoff wurde auf Grund erfolgreicher Tierversuche während des vergangenen Jahres in nahezu 30 humanen Operationen mit Erfolg eingesetzt, und zwar in den durch direkte chirurgische Erschliessung unlösbaren Fällen der angeführten Krankheiten. Unsere Erfahrungen haben eindeutig bewiesen, dass das in dem an die Zielstelle beförderten Kautschukballon bleibende vernetzte Silikonprodukt eine endgültige Verschliessung gewährleistet. Mit Hilfe des Kontrastschattens kann die Position des Ballons selbst nach Jahren mittels einer einfachen Röntgenaufnahme ermittelt und auch die Lage der polymerisierten Kautschukmasse bei freier Embolisierung bestimmt werden. Unsere wiederholten Untersuchungen bestätigten, dass der verwendete Silikonstoff – gleich anderen in der Chirurgie verbreiteten Silikonen (z.B. Shuntröhre zwischen Gehirnkammer und Herz, Gelenk- und

sonstige Plastiken, stomatologische Stoffe usw.) – für den menschlichen Organismus völlig unschädlich, untoxisch ist und keine histologische Reaktion infektiöser oder pathologischer Natur hervorruft. Es ist im wesentlichen diese Eigenschaft, die die Applikation der bereits erwähnten Methode der freien Embolisierung ermöglicht.

Die im beschriebenen Heilbehelf verwendete Silikonkautschuk-Mischung kann z.B. von folgender Zusammensetzung sein:

Beispiel 1

10 g Dimethyl-polysiloxan-α, ω-diol; Viskosität von 50 bis 100 mPas

1 g gepulverter, durch einen Sieb von 0,65 µm gesiebter, getrockneter Röntgenkontraststoff (Uromiro)

Das Gemisch wird in einem Homogenisierungsgerät sehr fein verarbeitet und vor der chirurgischen Anwendung in Ampullen sterilisiert. Desgleichen wird die zur gegebenen Masse benötigte Katalysatormenge sterilisiert und in Ampullen gefüllt. In diesem Fall handelt es sich um 1,5 $cm^3$ des Katalysators T-5 der Firma Wacker. Durch die Vermischung der beiden Komponenten in diesem Verhältnis wird im Laufe der Polymerisierung 8 Minuten «Topfzeit» und 15 Minuten Bindungszeit gesichert. Nach 15–25 Minuten kann der Katheter abgetrennt werden.

Beispiel 2

Eine andere Komponente A hat folgende Zusammensetzung:

10 g Dimethyl-polysiloxan-α, ω-diol; Viskosität 100 mPas

2 g nach Beispiel 1 vorbereiteter Röntgenkontraststoff

2 g Methylsilikonöl auf Dimethyl-polysiloxan-Basis; Viskosität 19 mPas

Die zur obigen Komponente benötigte Komponente B ist nach Qualität und Quantität gleich der vorherigen Angaben.

Beispiel 3

Komponente A:

2,5 g Dimethyl-polysiloxan-α, ω-diol; Viskosität 100 mPas

2,5 g $D_4$; Viskosität 4–5 mPas

0,75 g Bis-jodmethyl-tetramethyl-dissiloxan (flüssiger jodhaltiger Röntgenkontraststoff)

Die Mischung wird in einem Homogenisierungsgerät fein verarbeitet, nachher auf die übliche Weise in Ampullen gefüllt und sterilisiert. Als Komponente B kann der zu stomatologischen Zwecken empfohlene Flüssigkatalysator T-5 oder T-1 der Firma Wacker in der Menge von 1 $cm^3$ vorteilhaft verwendet werden.

Im folgenden wird auf einige klinische Anwendungen der Mischung, bzw. des Heilbehelfs laut Erfindung hingewiesen.

Es wurde unter Verwendung der erfindungsgemässen Mischung, bzw. des Heilbehelfs eine Operation an einem Patienten mit Schädelbasisfraktur vorgenommen. Der Bruch hatte die das Gehirn versehende rechtsseitige Hauptader beschädigt und die Bildung einer, eine schmerzhafte Augenblutung verursachenden, arteriovenösen Aderfistel herbeigeführt, die das Sehvermögen des rechten Auges gefährdete. Die Lösung mit traditionellen chirurgischen Methoden und mehreren Eingriffen versprach keine sichere Heilung, zudem muss hierbei die erhöhte Gefahr von Komplikationen mit in Kauf genommen werden. Unter Anwendung der Mischung und des Heilbehelfs laut Erfindung wurden ohne direkte Erschliessung zwei, mit Silikon gefüllte, abgetrennte Ballonköpfe mit dem durch die Halspunktion der Hauptader eingeführten Ballonkatheter in die Aderfistel befördert und diese mit Erfolg verschlossen. Der Patient heilte symptomfrei aus.

An einem Patienten mit Gehirnblutung wurde ein die rechtsseitige Hemisphäre des Grosshirns vollkommen ausfüllendes arteriovenöses Angiom als Quelle der Blutung nachgewiesen. Die direkte chirurgische Lösung des Angioms war wegen der Dimensionen und der Lage unmöglich. Daher wurden in drei Stufen drei grössere, das Angiom nährende Adern mit Hilfe von silikongefüllten Ballonen verschlossen, wonach die sich inzwischen wiederholenden Blutungen aufhörten, das Angiom sich auf einen Drittel des früheren Volumens zusammenzog und der Zustand des Kranken sich allmählich besserte. Ein Halbjahr nach der Operation war der Patient gehfähig, selbstversorgend, geistig geordnet.

Das Mittel, bzw. die Mischung laut Erfindung kann auch bei solchen Operationen mit Vorteil angewendet werden, wo zwar eine direkte Erschliessung vorgenommen wird, aber beim chirurgischen Eingriff mit einer starken Blutung gerechnet werden muss. In diesem Fall kann vor der direkten Erschliessung eine Embolisation angestellt werden, um die wichtigen Aderwege vorübergehend zu verschliessen. Nach der Beendigung der Operation wird dann der embolisierende Ballon auf einfache Weise entfernt.

**Patentansprüche**

1. Zur Anwendung in der endovasculären und allgemeinen Chirurgie geeignete, vorteilhaft einen Röntgenschatten gebende Silikonkautschuk-Mischung, dadurch gekennzeichnet, dass sie aus einem niederviskosen linearen Polysiloxan, nämlich einem Dialkyl-, Alkylaryl-, Alkenylalkyl- oder Diarylpolysiloxan mit Hydroxyl-, Alkoxy-, Acyloxy- oder Amino-Gruppen als reaktiven Endgruppen, einem niederviskosen cyclischen Diniederalkyl-polysiloxan und/oder aus einem für den menschlichen Organismus unschädlichen siliziumorganischen oder nicht metallischen jodhaltigen organischen Röntgenkontraststoff, sowie gegebenenfalls aus einem Methylsilikon besteht, wobei die Mischung eine Viskosität von 10 bis 100 mPas besitzt.

2. Mischung nach Anspruch 1, dadurch gekennzeichnet, dass sie als lineares Polysiloxan das lineare Dimethylpolysiloxan- α,ω-diol enthält.

3. Mischung nach Anspruch 1 oder 2, dadurch gekennzeichnet, dass sie als cyclisches Diniederalkylpolysiloxan Oktamethyl-cyclo-tetrasiloxan enthält.

4. Mischung nach Anspruch 1, dadurch gekennzeichnet, dass sie als Röntgenkontraststoff Bis-jodmethyl-tetramethyl-disiloxan enthält.

5. Heilbehelf, dadurch gekennzeichnet, dass er eine Silikonkautschuk-Mischung laut einem der Ansprüche 1 bis 4 als Komponente A, einen bekannten, zur medizinischen Anwendung geeigneten Vernetzungskatalysator als Komponente B, sowie einen Mikrokatheter und gegebenenfalls einen am Ende des Katheters angebrachten Gummiballon enthält.

**Claims**

1. A silicone rubber mixture suitable for application in endovascular and general surgery and advantageously serving as a contrast medium in the roentgenography, characterized in that it consists of a linear polysiloxane of low viscosity, i.e. a dialkyl-, alkylaryl-, alkenylalkyl- or diarylpolysiloxane having as reactive end-groups hydroxyl, alkoxy, acyloxy or amino groups, a cyclic di-lower-alkyl-polysiloxane having low viscosity and/or a siliceous-organic or non-metallic iodiferous organic radiopaque medium being harmless to the human organism, and, optionally, a methylsilicone, the mixture having a viscosity of from 10 to 100 mPas.

2. A mixture according to claim 1, characterized in that it contains as the linear polysiloxane linear dimethyl-polysiloxane-α, ω-diol.

3. A mixture according to one of claims 1 or 2, characterized in that it contains as the cyclic di-lower-alkyl-polysiloxane octamethyl-cyclo-tetrasiloxane.

4. A mixture according to claim 1, characterized in that it contains as the radiopaque medium bis-iodomethanetetramethyl-disiloxane.

5. A healing aid, characterized in that it contains a silicone rubber mixture according to any of the claims 1 to 4 as component A, a known cross-linking catalyst suitable for medical application as component B, a microcatheter, and, optionally, a rubber balloon placed at the end of the catheter.

**Revendications**

1. Mélange à base de caoutchouc de silicone produisant avantageusement une ombre radiologique, susceptible pour application dans le domaine de la chirurgie endovasculaire et générale, caractérisé en ce qu'il est constitué par un polysiloxane linéaire de faible viscosité, à savoir par un dialkylpolysiloxane, un alkylarylpolysiloxane, un alkénylalkylpolysiloxane ou un diarylpolysiloxane comprenant en tant que groupes terminaux réactifs des groupes hydroxy, alkoxy, acyloxy ou amino, par un di-(alkyl-inf)polysiloxane cyclique de faible viscosité et/ou par une substance de contraste radiologique, organique non métallique ou silico-organique renfermant de l'iode, non nocive pour l'organisme humain, ainsi que, le cas échéant, par une méthylsilicone, ledit mélange présentant une viscosité de 10 à 100 mPas.

2. Mélange selon la revendication 1, caractérisé en ce qu'il renferme, en tant que polysiloxane linéaire, du diméthyl-polysiloxane-alpha-oméga-diol linéaire.

3. Mélange selon la revendication 1 ou 2, caractérisé en ce qu'il renferme, en tant que di-(alkyl-inf)polysiloxane cyclique de l'octaméthyl-cyclo-tétrasiloxane.

4. Mélange selon la revendication 1, caractérisé en ce qu'il renferme, en tant que substance de contraste radiologique, du bis-iodométhyl-tétraméthyl-disiloxane.

5. Dispositif médical auxiliaire, caractérisé en ce qu'il comporte un mélange à base de caoutchouc de silicone selon l'une des revendications 1 à 4, en tant que composant A, un catalyseur de réticulation connu en soi, se prêtant à des applications médicales, en tant que composant B, ainsi qu'un microcathéter et, le cas échéant, un ballon de caoutchouc disposé à l'extrémité dudit cathéter.